# EUROPÄISCHE PATENTANMELDUNG

(11) **EP 1 072 885 A2**
(43) Veröffentlichungstag der Anmeldung: **31.01.2001**
(21) Anmeldenummer: 00113310.7
(22) Anmeldetag: 23.06.2000
(51) Int. Cl.: G01N 27/22

(54) **Einrichtung zur Messung der Feuchtigkeit von Erntegut**

(30) Priorität: 24.07.1999 DE 19934881
(71) Anmelder: DEERE & COMPANY, Moline, Illinois 61265-8098 (US)
(72) Erfinder: Rode, Hans-Jürgen, 24955 Harrislee (DE); Rutz, Arnold, 66482 Zweibrücken (DE)
(74) Vertreter: Holst, Sönke, Dr.

(57) **Zusammenfassung**

Die Erfindung bezieht sich auf eine Einrichtung zur Messung der Feuchtigkeit von Erntegut, mit einem Meßkondensator (52), der zwei Elektroden (78, 80, 82) aufweist, zwischen denen Erntegut einbringbar ist, und der mit einer Induktivität (104) zu einem Resonanzkreis (106) geschaltet ist. Um eine präzise Messung zu ermöglichen, wird vorgeschlagen, daß der Resonanzkreis (106) mit einem Meßsignal in Form einer Wechselspannung variabler Frequenz beaufschlagt wird, und daß ein Parameter des Resonanzkreises (106) bei unterschiedlichen Frequenzen erfaßt und zur Bestimmung der Feuchtigkeit herangezogen wird.

## Beschreibung

Die Erfindung betrifft eine Einrichtung zur Messung der Feuchtigkeit von Erntegut, mit einem Meßkondensator, der zwei Elektroden aufweist, zwischen denen Erntegut positionierbar ist, und der mit einer Induktivität zu einem Resonanzkreis geschaltet ist, und ein Verfahren zur Messung der Feuchtigkeit von Erntegut.

Aus der US 4 584 522 A ist eine Einrichtung zur Feuchtigkeitsmessung von Erntegut bekannt geworden, die nach einem kapazitiven Meßprinzip arbeitet. Ein Behälter wird mit einem Erntegut unbekannter Feuchtigkeit gefüllt und anhand der Änderung der Kapazität einer Elektrode in Form einer Kondensatorplatte gegenüber einer zweiten Elektrode kann die Feuchtigkeit des Guts bestimmt werden. Trockenes Gut bewirkt aufgrund der verhältnismäßig geringen Dielektrizitätszahl ε nur eine kleinere Kapazitätsänderung gegenüber einem leeren Behälter als feuchtes Gut mit hoher Dielektrizitätszahl. Bei der Messung wird der Kondensator mit unbekannter Kapazität C mit einer vorbestimmten Spannung aufgeladen, und nach Abschalten der Ladungsquelle über einen Widerstand R entladen, wobei die nach einer gewissen Zeit noch am RC-Glied anliegende Spannung ein Maß für die Kapazität C ist.

In der DE 1300316 B ist eine Vorrichtung zur kontinuierlichen Messung von Schüttgut beschrieben, bei der die dielektrischen Verluste des Meßkondensators als Maß für den Wassergehalt des Meßgutes dienen. Es wird also ein parasitärer (ohmscher) Widerstand des mit hochfrequenter Wechselspannung beaufschlagten Kondensators gemessen.

Weiterhin sind auch Resonanzmeßschaltungen für Meßeinrichtungen zur Feuchtigkeitsbestimmung vorgeschlagen worden. Bei einer von W. Lück in dem Buch Feuchtigkeit, Grundlagen, Messen, Regeln, S. 221 ff (Verlag R. Oldenbourg, München, 1964), beschriebenen Meßeinrichtung gattungsgemäßer Art wird die Kapazität eines Meßkondensators durch Messung der Spannung an einem LC-Parallelschwingkreis bei einer Frequenz, an der die Flankensteilheit des Schwingkreises maximal ist, bestimmt. Alternativ wird der Meßkondensator als frequenzbestimmendes Element in einen Oszillator eingefügt.

Als nachteilig ist bei den bekannten Feuchtigkeitsmeßeinrichtungen anzusehen, daß es bei den Messungen auf den Absolutwert einer am Meßkondensator anliegenden Spannung ankommt, wobei an den Meßkondensator eine Meßspannung in Form einer Gleichspannung oder Wechselspannung konstanter Frequenz angelegt wird. Der angezeigte Feuchtigkeitsmeßwert hängt daher direkt von der Meßspannung ab. Es ist jedoch problematisch, die Meßspannung, mit der der Kondensator beaufschlagt wird, zur Erzielung reproduzierbarer, verläßlicher Ergebnisse konstant zu halten. Daher sind die Meßgeräte relativ unpräzise.

Das der Erfindung zugrunde liegende Problem wird darin gesehen, die Genauigkeit einer Einrichtung zur Messung der Feuchtigkeit von Erntegut zu verbessern.

Diese Aufgabe wird erfindungsgemäß durch die Lehre der Patentansprüche 1 und 11 gelöst, wobei in den weiteren Patentansprüchen Merkmale aufgeführt sind, die die Lösung in vorteilhafter Weise weiterentwickeln.

Der Kerngedanke der Erfindung besteht darin, die Frequenz des wechselstromförmigen Meßsignals zu variieren, mit dem der Resonanzkreis beaufschlagt wird, und bei unterschiedlichen Frequenzen einen Parameter des Resonanzkreises zu erfassen. Da der frequenzabhängige Parameter des Resonanzkreises (unter anderem) von der Kapazität des Meßkondensators abhängt, besteht auch ein Zusammenhang mit der Feuchtigkeit des Meßgutes, die anhand des gemessenen Parameters bestimmt werden kann.

Auf diese Weise, d. h. durch das Variieren der Frequenz und das Erfassen des frequenzabhängigen Parameters erreicht man, daß der Absolutwert eines gemessenen Signals keine Fehlerquelle mehr darstellt, da nur die Frequenzabhängigkeit, jedoch nicht der Absolutwert des Signals für die weitere Auswertung relevant ist. Die erfindungsgemäße Meßeinrichtung arbeitet daher sehr präzise.

Als erfaßter Parameter des Resonanzkreises kommt primär seine Impedanz in Frage. Denkbar wäre aber auch, die Phasenverschiebung zwischen einem Meßsignal, das dem Schwingkreis über einen hochohmigen Widerstand zugeführt wird, und der am Schwingkreis anliegenden Spannung als Parameter des Resonanzkreises zu erfassen. Die Phasenverschiebung ist ebenfalls von der Kapazität des Meßkondensators abhängig und erlaubt eine Bestimmung der Feuchtigkeit des Meßgutes.

Zur Erfassung der Impedanz des Resonanzkreises kann die an ihm anliegende Spannung, bzw. ihre Amplitude, gemessen werden, wobei er Bestandteil eines Spannungsteilers ist. Das Meßsignal wird dem ersten Anschluß des Resonanzkreises über einen Widerstand oder Kondensator zugeführt, und der zweite Anschluß des Resonanzkreises ist mit einem zweiten Anschluß der Meßsignalquelle verbunden. Am Resonanzkreis kann dann eine Spannung erfaßt werden, die von seiner Impedanz abhängt.

Die Messung der Impedanz des Resonanzkreises kann insbesondere derart erfolgen, daß er durch eine Konstantstromquelle mit dem Meßsignal beaufschlagt wird. Als Konstantstromquelle kann insbesondere eine Spannungsquelle dienen, die über einen hinreichend großen Widerstand mit dem Resonanzkreis verbunden ist, so daß der vom Resonanzkreis aufgenommene Strom konstant und von seiner Impedanz unabhängig ist; dann ist die am Resonanzkreis anliegende Spannung ein direktes Maß für seine Impedanz. Alternativ kann auch ein Strom gemessen werden, der durch den Resonanzkreis fließt. Dazu kann ein Shunt-Widerstand dienen, an dem eine zum Strom proportionale Spannung abgegriffen wird, die ebenfalls ein Maß für die Impedanz des Resonanzkreises ist. In diesem Fall wird der Resonanzkreis durch eine Konstantspannungsquelle mit dem Meßsignal beaufschlagt.

Der Resonanzkreis ist vorzugsweise ein Parallelschwingkreis. Der Vorteil liegt gegenüber einem Reihenschwingkreis, der grundsätzlich auch verwendbar wäre, in der Unabhängigkeit der Resonanzfrequenz von parallelgeschalteten parasitären Widerständen, die durch Oberflächenfeuchtigkeit des Meßguts entstehen können.

Anhand der Frequenzabhängigkeit des erfaßten Parameters wird vorzugsweise die Resonanzfrequenz des Resonanzkreises bestimmt, die ihrerseits bei bekannter und fester Induktivität L ein Maß für die Kapazität C des Meßkondensators ist. Letztere erlaubt wiederum, die Dielektrizitätszahl des im Meßkondensator enthaltenen Ernteguts zu bestimmen, die von der Feuchtigkeit des Ernteguts abhängt. Auch letztere kann daher unschwer bestimmt werden. Die Resonanzfrequenz liegt bei der Frequenz, bei der die Impedanz des Resonanzkreises maximal (Parallelschwingkreis) bzw. minimal (Reihenschwingkreis) ist; es sind jedoch auch andere Kriterien zur Erkennung der Resonanzfrequenz denkbar, wie die Phasenverschiebung. Bei der Auswertung ist beliebig, ob bei der Berechnung der Feuchtigkeit, also softwareseitig, in zwischengeordneten Rechenschritten die Resonanzfrequenz und die Kapazität des Kondensators bzw. die Dielektrizitätszahl ε explizit bestimmt werden, oder ob die Feuchtigkeit direkt anhand der gemessenen Parameter oder einer in irgendeiner Weise daraus abgeleiteten Resonanzfrequenz, Kapazität, Dielektrizitätszahl oder einem beliebigen davon abhängigen Wert bestimmt wird.

Da auch die Temperatur, bei der die Messung erfolgt, wie auch die Art des Ernteguts den Zusammenhang zwischen der Dielektrizitätszahl ε und der Feuchtigkeit beeinflussen, wird die Feuchtigkeit vorzugsweise anhand einer Tabelle bestimmt, in der für die jeweilige Temperatur, die mittels eines geeigneten Sensors erfaßt wird, und/oder die jeweilige Art des Ernteguts (insbesondere Getreideart), die vom Benutzer eingebbar ist, einer gemessenen Resonanzfrequenz eine Feuchtigkeit zugeordnet ist. Anstelle der resonanzfrequenzabhängigen Feuchtigkeit kann in der Tabelle auch die Feuchtigkeit in Abhängigkeit von der Kapazität C, der Dielektrizitätszahl ε oder einem beliebigen anderen, in Zusammenhang mit einer dieser Größen stehenden Wert abgelegt sein. Derartige Tabellen sind durch Messungen mit bekannten Parametern kalibrierbar.

Weiterhin kann die Frequenz des Meßsignals, also der Wechselspannung, mit der der Resonanzkreis beaufschlagt wird, kontinuierlich oder in Stufen variiert werden. Ein kontinuierliches Durchlaufen (Wobbeln) des Frequenzbereichs kann durch analoge oder digitale Schaltungstechnik mittels eines dreieck- oder sägezahnförmigen Steuersignals erzeugt werden, das einem spannungsgesteuerten Oszillator (Voltage Controlled Oscillator, VCO) zugeführt wird. Das stufenförmige Durchlaufen des Frequenzbereichs kann durch eine treppenstufenförmige Steuerspannung des VCO erzielt werden, die ebenfalls durch analoge oder digitale Schaltungstechnik realisierbar ist. Auch eine rein digitale Erzeugung des Meßsignals unter Verwendung eines Digital-Analog-Wandlers ist denkbar. Es wird ein vorzugsweise vorbestimmter Frequenzbereich durchlaufen, in dem die zu erwartende Resonanzfrequenz des Resonanzkreises liegt. Denkbar wären auch mehrere Durchläufe, von denen ein erster in relativ großen Stufen erfolgt, und ein zweiter nur einen kleineren Bereich um die im ersten Durchlauf grob herausgefundene Resonanzfrequenz abdeckt, aber wesentlich kleinere Stufen verwendet, so daß die Resonanzfrequenz präzise erfaßt wird.

Außer der Bestimmung der Resonanzfrequenz erlaubt das erfindungsgemäße Meßverfahren auch die Breite der Resonanzkurve des Resonanzkreises zu erfassen. Sie ist um so breiter, je größer die Dämpfung durch parasitäre Widerstände ist, die parallel zum Resonanzkreis geschaltet sind. Derartige parasitäre Widerstände sind in der Regel durch Oberflächenfeuchtigkeit des Meßguts bedingt, die einen ohmschen Widerstand zwischen den Elektroden des Meßkondensators realisiert. Es bietet sich daher an, auch die Breite der Resonanzkurve zwecks Bestimmung der Oberflächenfeuchtigkeit auszuwerten. Dabei ist beliebig, welche Breite der Resonanzkurve erfaßt wird (Basisbreite, Halbwertsbreite, etc.). Die gemessene Oberflächenfeuchte kann mittels einer Anzeigeeinrichtung wiedergegeben werden. Bei Überschreiten eines Schwellenwertes kann eine Fehlermeldung abgegeben werden, da das Erntegut in diesem Fall offenbar zu feucht ist, um geerntet zu werden.

Die Erfindung findet vorzugsweise in landwirtschaftlichen Maschinen Verwendung, insbesondere in Mähdreschern oder Feldhäckslern, in denen sie die Feuchtigkeit des geernteten Gutes (in der Regel Getreide) erfaßt. Die gemessenen Feuchtigkeitswerte, wie auch ggf. Oberflächenfeuchtigkeiten, kennen zur Ertragskartierung georeferenziert abgespeichert oder durch Datenfernübertragung zu einer Zentrale übertragen werden.

In der Zeichnung ist ein nachfolgend näher beschriebenes Ausführungsbeispiel der Erfindung dargestellt. Es zeigt:
- Fig. 1: eine Seitenansicht eines landwirtschaftlichen Axialmähdreschers,
- Fig. 2: einen Schnitt durch einen Meßkondensator einer Einrichtung zur Messung der Feuchtigkeit des Ernteguts, und
- Fig. 3: ein Prinzipschaltbild der Einrichtung zur Messung der Feuchtigkeit, und
- Fig. 4: einen Ausschnitt aus Figur 3 mit einer alternativen Spannungsteilerschaltung.

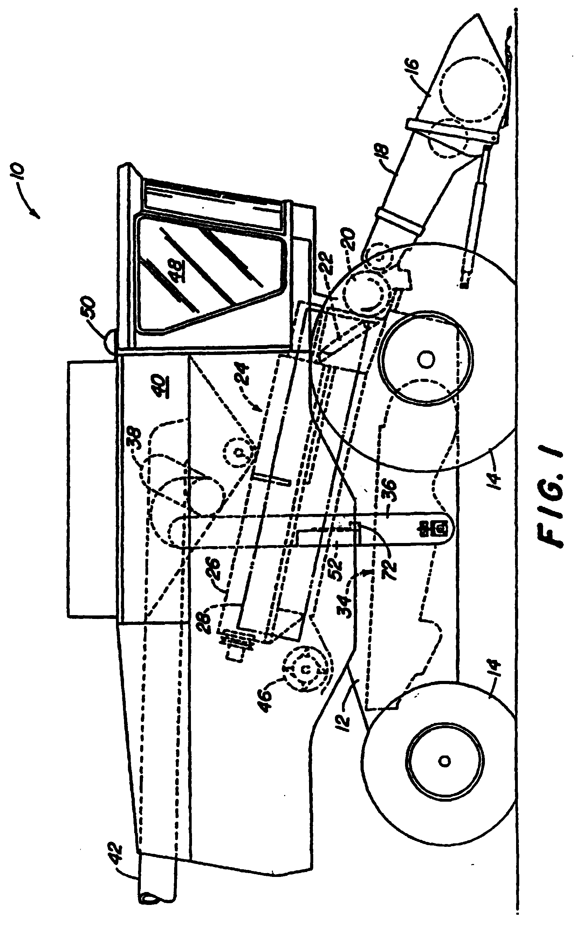

Figur 1 zeigt einen landwirtschaftlichen Mähdrescher 10 mit einem Fahrgestell 12 und sich von diesem erstreckenden Bodenlaufrädern 14. Eine Erntegutbergungsvorrichtung 16 wird verwendet, Erntegut aufzunehmen und es einem Schrägförderer 18 zuzuführen. Das Erntegut wird vom Schrägförderer 18 einer Leittrommel 20 zugeführt. Die Leittrommel 20 leitet das Erntegut nach oben durch einen Einlaßübergangsbereich 22 an eine Axialtrennvorrichtung 24 weiter. Obwohl die Erfindung am Beispiel eines Axialmähdreschers beschrieben wird, kann sie auch an anderen Mähdreschern mit einem Elevator für reines Korn, wie konventionellen Strohschüttlermaschinen, verwendet werden.

Die Axialtrennvorrichtung 24 umfaßt ein Rotorgehäuse 26 und einen im Rotorgehäuse 26 angeordneten Rotor 28. Das geerntete Gut tritt durch den Einlaßübergangsbereich 22 in das Rotorgehäuse 26 ein. Die Axialtrennvorrichtung 24 drischt und trennt das geerntete Gut. Korn und Spreu fallen durch Roste bzw. Dreschkörbe am Boden der Axialtrennvorrichtung 24 in ein Reinigungssystem 34. Das Reinigungssytem 34 entfernt die Spreu und führt das saubere Korn einem Kornelevator 36 zu, der es wiederum einer Verteilerschnecke 38 zuführt. Die Verteilerschnecke 38 legt das saubere Korn in einem Korntank 40 ab. Das saubere Korn im Korntank 40 kann durch eine Entladeschnecke 42 in einen Anhänger oder Lastwagen entladen werden. Gedroschenes, vom Korn befreites Stroh wird aus der Axialtrennvorrichtung 24 durch einen Auslaß heraus zu einer Abgabetrommel 46 geführt. Die Abgabetrommel 46 wirft das Stroh am rückwärtigen Ende des Mähdreschers 10 aus.

Die Bedienung des Mähdreschers 10 erfolgt von einer Fahrerkabine 48 aus. Ein Empfänger 50 zum Empfang von GPS (Global Positioning System) - Signalen ist über der Fahrerkabine 48 angebracht.

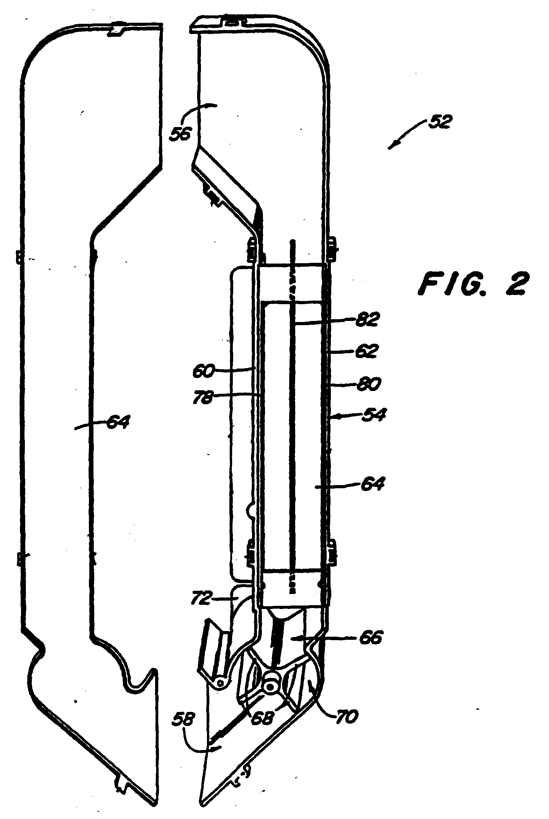

An einer Seite des Kornelevators 36 ist der Meßkondensator 52 einer Einrichtung zur Messung der Feuchtigkeit des Korns befestigt. Der Meßkondensator 52 umfaßt eine vertikale Kammer 54 mit einem Einlaß 56 zur Aufnahme sauberen Korns aus dem Kornelevator 36 und einen Auslaß 58, durch den das Korn wieder in den Kornelevator 36 verbracht wird. Die Kammer 54 selbst hat eine erste Wand 60, die dem Kornelevator 36 benachbart und parallel dazu verläuft. Parallel und beabstandet von der ersten Wand 60 verläuft eine zweite Wand 62 der Kammer 54. Seitenwände 64 verbinden die erste Wand 60 und die zweite Wand 62. Die Kammer 54 ist aus einem nicht-leitenden Material wie Kunststoff hergestellt. Eine der Seitenwände 64 ist abnehmbar und in der Figur 2 in abgenommener Stellung wiedergegeben.

Eine Einrichtung zur Steuerung des Flusses des Korns durch den Meßkondensator 52 umfaßt ein Paddelrad 66, das unmittelbar stromauf des Auslasses 58 angeordnet ist. Das Paddelrad 66 hat vier flexible Gummipaddel 68, die sich über die ganze Höhe der Kammer 54 zwischen den Seitenwänden 64 erstrecken. Ein zylindrischer Bereich 70 ist in der Kammer 54 zur Aufnahme des Paddelrades 66 geformt. Das Paddelrad 66 wird durch einen Elektromotor 72 angetrieben, der so betrieben wird, daß das Paddelrad 66 den Fluß des Korns durch die Kammer 54 derart steuert, daß eine für eine Feuchtigkeitsmessung hinreichende Kornmenge darin vorhanden ist. Eine entsprechende Steuerung ist in der CA 2182989 A offenbart, deren Inhalt durch Verweis hierin aufgenommen wird.

Die Kammer ist auch mit Elektroden zur Kapazitätsmessung ausgestattet, die erste, zweite und dritte Metallplatten 78, 80 und 82 umfassen. Die ersten zwei Metallplatten 78, 80 sind benachbart und parallel zu den ersten und zweiten Wänden 60, 62 angeordnet und elektrisch miteinander verbunden. Die dritte Metallplatte 82 verläuft parallel zu den ersten zwei Metallplatten 78, 80 und ist in der Mitte zwischen ihnen angeordnet. Sauberes, zwischen den Metallplatten 78, 80, 82 hindurchströmendes Korn ist ein dielektrisches Material, das die Kapazität zwischen ihnen beeinflußt, die von der Feuchtigkeit des Materials abhängt.

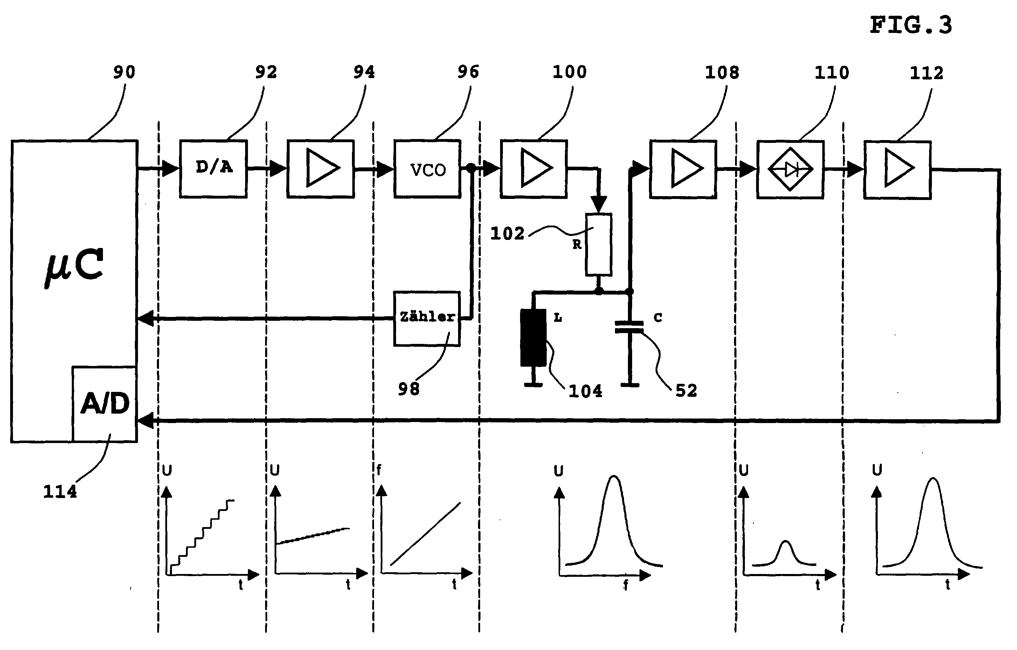

In Figur 3 ist ein Blockschaltbild einer Einrichtung zur Bestimmung der Feuchtigkeit des Korns wiedergegeben. Sie umfaßt einen Mikrocontroller 90 (anstelle dessen auch ein Mikroprozessor verwendet werden könnte) und einen vom Mikrocontroller 90 gesteuerten Digital-Analog-Wandler 92, dessen Ausgangssignal einem Anpassungsverstärker 94 zugeführt wird. Die Aufgabe des Anpassungsverstärkers 94 besteht darin, die Ausgangsspannung des Digital-Analog-Wandlers 92 in der Höhe derart anzupassen, daß sie zur Steuerung eines spannungsgesteuerten Oszillators (VCO) 96 geeignet ist. Der Ausgang des spannungsgesteuerten Oszillators 96 ist mit dem Eingang eines zweiten Verstärkers 100 und dem Eingang eines Frequenzzählers 98 verbunden.

Der Mikrocontroller 90 beaufschlagt den Digital-Analog-Wandler 92 mit einem (digitalen) Signal, das letzteren veranlaßt, an seinem Ausgang eine treppenstufenförmige Spannung zu liefern. Der zeitabhängige Spannungsverlauf am Ausgang des Digital-Analog-Wandlers 92 ist in Figur 3 unter dem Digital-Analog-Wandler 92 wiedergegeben. Der Anpassungsverstärker 94 liefert eine in Figur 3 unter ihm wiedergegebene Ausgangsspannung mit flacherem Verlauf, die einen Frequenzbereich des spannungsgesteuerten Oszillators 96 bewirkt, der den Resonanzbereich des Resonanzkreises 106, in dem sich der Meßkondensator 52 befindet, hinreichend abdeckt. Am Ausgang des spannungsgesteuerten Oszillators 96 steht eine sinusförmige Wechselspannung zur Verfügung, deren Frequenz mit der Zeit sägezahnförmig ansteigt. Dieses sog. Wobbel-Signal ist in Figur 3 unter dem spannungsgesteuerten Oszillator 96 wiedergegeben.

Der Frequenzzähler 98 liefert dem Mikrocontroller 90 wiederum ein digitales Signal, das eine Information über die jeweilige Frequenz enthält. Es wäre zwar denkbar, einen Nominalwert der Frequenz anhand des vom Mikrocontroller 90 dem Digital-Analog-Wandler 92 zugeführten Signals zu erfassen, was aufgrund der Temperaturabhängigkeit der Spannungs-Frequenz-Charakteristik des spannungsgesteuerten Oszillators 96 jedoch relativ ungenau wäre. Durch das Erfassen der jeweiligen Frequenz mittels des Frequenzzählers 98 führt man dem Mikrocontroller 90 einen präziseren, temperaturunabhängigen Wert für die Frequenz zu.

Der Verstärker 100, der in der Regel ein Spannungsfolger ist, verstärkt das Ausgangssignal des spannungsgesteuerten Oszillators 96 und führt das verstärkte Signal einem mehr oder weniger hochohmigen Widerstand 102 zu, anstelle dessen auch ein Kondensator mit geringer Kapazität verwendbar wäre. Der Verstärker 100 und der Widerstand 102 realisieren im Zusammenwirken mit einem aus einer Induktivität 104 und dem Kondensator 52 aufgebauten Resonanzkreis 106 einen Spannungsteiler. Das am 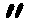 heißen" Ende des Resonanzkreises 106 anliegende Signal hängt von der Impedanz des Resonanzkreises 106 ab. Wählt man den Wert des Widerstands 102 hinreichend hoch, realisiert er eine Stromquelle, deren Ausgangsstrom konstant, d. h. von der Frequenz und der Impedanz des Resonanzkreises 106 unabhängig ist. Die Höhe der Spannung am Resonanzkreis 106 ist dann direkt proportional zu seiner Impedanz.

Der dem Verstärker 100 abgewandte Anschluß des Widerstands 102 ist mit dem Parallelresonanzkreis 106 verbunden. Dessen Kondensator 52 besteht aus den Metallplatten 78, 80, 82, von denen die dritte Metallplatte 82 mit dem Widerstand 102 verbunden ist, und sich die ersten zwei Metallplatten 78, 80 auf Massepotential befinden (bzw. umgekehrt). Am 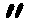 heißen" Ende des Resonanzkreises 106, d. h. am unten eingezeichneten Anschluß des Widerstands 102, liegt eine frequenzabhängige Spannung an, deren in Figur 3 unter dem Resonanzkreis 106 dargestellte Amplitude U₀ (die ein Maß für die Impedanz des Resonanzkreises 106 ist) bei der Resonanzfrequenz maximal ist. Die Resonanzfrequenz liegt bekannterweise bei 1/2π(LC)^{1/2}. Wegen der linearen Abhängigkeit der Frequenz von der Zeit (s. Kurve unter dem spannungsgesteuerten Oszillator 96) könnte die Amplitude statt in Abhängigkeit von der Frequenz auch in Abhängigkeit von der Zeit aufgetragen werden, ohne daß sich die Kurvenform ändern würde.

Der Verstärkungsfaktor des Verstärkers 100 kann in Ausführungsformen der Erfindung durch den Mikrocontroller 90 steuerbar sein. Diese Maßnahme erlaubt, die dem Schwingkreis 106 zugeführte Spannungsamplitude in Fällen, in denen eine starke Bedämpfung durch parasitäre Widerstände, die durch Oberflächenfeuchtigkeit des Guts bedingt sind, zu vergrößern, um ein zur Auswertung hinreichend großes Signal am Verstärker 108 zu erhalten.

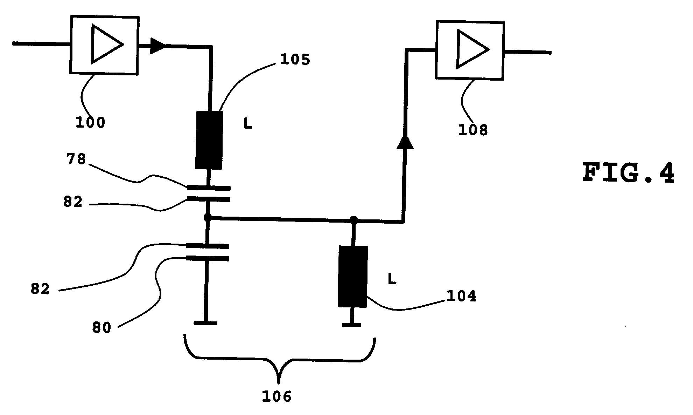

Die am Resonanzkreis 106 anliegende Spannung wird mittels eines dritten Verstärkers 108 (in der Regel ebenfalls ein Spannungsfolger) verstärkt, einem Gleichrichter 110 zugeführt, der sie gleichrichtet, und mittels eines vierten Verstärkers 112, der vorzugsweise auch ein Spannungsfolger ist, nochmals verstärkt. Am Ausgang des Gleichrichters 110 liegt eine zeitabhängige (Gleich-)Spannung an, die zur Amplitude der am Resonanzkreis anliegenden Spannung proportional ist. Sie ist unterhalb des Gleichrichters 110 in Figur 3 dargestellt. Diese Spannung wird durch den vierten Verstärker 112 verstärkt, dessen Ausgangssignal in Figur 4 unter ihm dargestellt ist, und schließlich dem Eingang eines in den Mikrocontroller 90 integrierten Analog-Digital-Wandlers 114 zugeführt.

Der Mikrocontroller 90 wird durch eine Software gesteuert, die in einem RAM oder ROM abgelegt ist. Eine Messung der Feuchtigkeit des geernteten Korns kann kontinuierlich oder auf eine Eingabe des Bedieners des Mähdreschers stattfinden. Beim Feuchtigkeitsmessen veranlaßt der Mikrocontroller 90, daß ein Frequenzbereich um die Resonanzfrequenz des Resonanzkreises 106 herum durchlaufen und anhand des Ausgangswertes des Analog-Digital-Wandlers 114 die Resonanzfrequenz bestimmt wird. Da die Kapazität des Kondensators 52 über die die Dielektrizitätzahl ε von der Feuchtigkeit des Korns abhängt, und wiederum die Resonanzfrequenz des Resonanzkreises 106 von der Kapazität abhängt, ist auf diese Weise eine einfache, aber präzise Messung der Feuchtigkeit des Korns möglich. Unter Verwendung einer Tabelle oder anderer abgespeicherter mathematischer Zusammenhänge wird anhand der gemessenen Resonanzfrequenz die Feuchtigkeit ermittelt. Die ermittelten Feuchtigkeitswerte können unter Verwendung der Positionssignale des GPS-Empfängers 50 georeferenziert abgespeichert oder anderweitig verwendet werden.

In Figur 4 ist eine abgewandelte Schaltung des Spannungsteilers der Figur 3 dargestellt, in der für den Resonanzkreis 106 nur das Metallplattenpaar 80, 82 verwendet wird. Die Induktivität 104 ist parallel zu diesem Plattenpaar 80, 82 geschaltet und die Spannung daran wird erfaßt, d. h. dem Verstärker 108 zugeführt. Am Ausgang des Verstärkers 100 ist eine zweite Induktivität 105, deren Größe mit der der Induktivität 104 übereinstimmt, angeschlossen. Der zweite Anschluß der Induktivität 105 ist mit der verbleibenden Metallplatte 78 verbunden. Im Ergebnis erhält man einen Spannungsteiler aus einem Reihenresonanzkreis (zweite Induktivität 105 und Metallplattenpaar 78, 82) und einem Parallelschwingkreis (Resonanzkreis 106 mit Induktivität 104 und Metallplattenpaar 80, 82), wobei an der mittleren Metallplatte 82 das frequenzabhängige Signal erfaßt wird. Diese Spannungsteilerschaltung hat eine größere Flankensteilheit als die in Figur 3 dargestellte, erlaubt also eine bessere Erkennung der Resonanzfrequenz. Selbstverständlich können die Anschlüsse der Metallplatten 78, 80 vertauscht werden.

## Patentansprüche

1. Einrichtung zur Messung der Feuchtigkeit von Erntegut, mit einem Meßkondensator (52), der zwei Elektroden (78, 80, 82) aufweist, zwischen denen Erntegut einbringbar ist, und der mit einer Induktivität (104) zu einem Resonanzkreis (106) geschaltet ist, dadurch gekennzeichnet, daß der Resonanzkreis (106) mit einem Meßsignal in Form einer Wechselspannung variabler Frequenz beaufschlagt wird, und daß ein Parameter des Resonanzkreises (106) bei unterschiedlichen Frequenzen erfaßt und zur Bestimmung der Feuchtigkeit herangezogen wird.

2. Einrichtung nach Anspruch 1, dadurch gekennzeichnet, daß der erfaßte Parameter von der Impedanz des Resonanzkreises (106) abhängt.

3. Einrichtung nach Anspruch 2, dadurch gekennzeichnet, daß eine Spannung am Resonanzkreis (106) erfaßt wird, der Bestandteil eines Spannungsteilers ist, der mit dem Meßsignal beaufschlagt wird.

4. Einrichtung nach Anspruch 3, dadurch gekennzeichnet, daß eine Spannung am Resonanzkreis (106) erfaßt wird, der durch eine Konstantstromquelle mit dem Meßsignal beaufschlagt wird, und/oder die Stärke des Stroms durch den Resonanzkreis (106), der durch eine Konstantspannungsquelle mit dem Meßsignal beaufschlagt wird.

5. Einrichtung nach einem der vorhergehenden Ansprüche, dadurch gekennzeichnet, daß der Resonanzkreis (106) ein Parallelschwingkreis ist.

6. Einrichtung nach einem der vorhergehenden Ansprüche, dadurch gekennzeichnet, daß anhand des erfaßten Parameters die Resonanzfrequenz des Resonanzkreises (106) ermittelt wird.

7. Einrichtung nach Anspruch 6, dadurch gekennzeichnet, daß die Feuchtigkeit anhand einer Tabelle bestimmt wird, in der Feuchtigkeitswerte in Abhängigkeit von der Resonanzfrequenz des Resonanzkreises (106) und vorzugsweise in Abhängigkeit von der Temperatur und/oder einer Art des Ernteguts, insbesondere einer Getreideart, abgelegt sind.

8. Einrichtung nach einem der vorhergehenden Ansprüche, dadurch gekennzeichnet, daß die Frequenz des Meßsignals kontinuierlich oder in Stufen über einen vorzugsweise vorbestimmten Frequenzbereich variiert wird.

9. Einrichtung nach einem der vorhergehenden Ansprüche, dadurch gekennzeichnet, daß zur Bestimmung der Oberflächenfeuchtigkeit des Meßguts die Breite der Resonanzkurve des Resonanzkreises (106) ausgewertet wird.

10. Einrichtung nach Anspruch 9, dadurch gekennzeichnet, daß bei Überschreiten eines Schwellenwerts der Oberflächenfeuchtigkeit eine Fehlermeldung gegeben wird.

11. Landwirtschaftliche Maschine, insbesondere Mähdrescher oder Feldhäcksler, mit einer Einrichtung nach einem der vorhergehenden Ansprüche.

12. Verfahren zur Messung der Feuchtigkeit von Erntegut, gekennzeichnet durch folgende Schritte:
Erntegut wird zwischen zwei Elektroden (78, 80, 82) eines Meßkondensators (52), der mit einer Induktivität (104) zu einem Resonanzkreis (106) geschaltet ist, eingebracht,
der Resonanzkreis (106) wird mit einem Meßsignal in Form einer Wechselspannung variabler Frequenz beaufschlagt,
bei unterschiedlichen Frequenzen ein Parameter des Resonanzkreises (106) erfaßt,
anhand der gemessenen Parameter wird die Feuchtigkeit berechnet.
